# EUROPEAN PATENT APPLICATION

(11) **EP 4 838 254 A1**
(43) Date of publication of application: **11.11.2026**
(21) Application number: 26176409.6
(22) Date of filing: 05.05.2026
(51) Int. Cl.: G01S 7/41, A61B 5/0507, G01S 7/35, G01S 7/292, G01S 13/02, G01S 13/87

(54) **TRANSFER LEARNING METHOD FOR DETERMINING HEART RATE BASED ON RADAR DATA**

(30) Priority: 06.05.2025 US 202563800802 P
(71) Applicant: Google LLC, Mountain View, CA 94043 (US)
(72) Inventor: GRUZEWSKA, Elzbieta, London, N1C 4AG (GB); DASWANI, Mayank, London, N1C 4AG (GB); RAO, Pooja, 1082 MD Amsterdam (NL); RUDRAPATNAV, Pramod, Mountain View, California, 94043 (US); BAUR, Sebastien Jean Jean-Paul, Mountain View, California, 94043 (US); BAUGH, Matthew, London, N1C 4AG (GB)
(74) Representative: Maikowski & Ninnemann Patentanwälte Partnerschaft mbB

(57) **Abstract**

A computer-implemented method for transferring a machine-learned model trained on a first dataset of a first type of radar data to a second dataset of a second type of radar data to leverage learnings from the first and second datasets is disclosed. The first dataset is larger than the second dataset. The computer-implemented method includes (a) initializing the machine-learned model, (b) training the machine-learned model on the first dataset of the first type of radar data and biometric ground truth data, after (b), then (c) training the machine-learned model on the second dataset of the second type of radar data and the biometric ground truth data, and after (c), then (d) outputting the machine-learned model for use in predicting one or more biometric parameters of a user from the radar data.

## Description

### FIELD

The present disclosure relates generally to machine learning processes and machine-learned devices and systems. More particularly, the present disclosure relates to a computer-implemented method for transferring a machine-learned model trained on a larger first dataset of a first type of radar data to a second dataset of a second type of radar data to leverage learnings from the first and second datasets.

### BACKGROUND

Contactless vital sign monitoring has gained significant attention in recent years, driven by the increasing need for unobtrusive and continuous health monitoring solutions. Radar technology, in particular, has emerged as a promising approach for remote heart rate (HR) detection. Unlike traditional contact-based methods, radar offers the advantage of non-contact sensing, eliminating the need for physical attachments to the body.

Several radar-based HR monitoring systems have been developed, employing various radar modalities. Among these, millimeter-wave Frequency-Modulated Continuous Wave (mm-wave FMCW) radar and impulse-radio ultra-wideband (IR-UWB) radar have garnered considerable interest. Mm-wave FMCW radar offers high range resolution and sensitivity, enabling precise detection of subtle chest movements associated with cardiac activity. IR-UWB radar, on the other hand, provides wider bandwidth and lower power consumption, making it suitable for portable and battery-powered devices.

Despite the potential of radar-based HR monitoring, existing technologies face several limitations. One challenge is the susceptibility to interference from respiratory motion, which can mask the weaker cardiac signals. While signal processing techniques such as band-pass filtering and adaptive filtering have been employed to mitigate this issue, they often struggle to completely eliminate respiratory artifacts without distorting the HR signal. Furthermore, most existing radar-based HR monitoring systems are designed for specific radar modalities and environmental conditions, limiting their adaptability to diverse deployment scenarios. Current systems are also limited by the amount of data needed to build effective models that can extract heart rate readings.

Therefore, there is a need for more robust and versatile radar-based HR monitoring techniques that can overcome the limitations of prior art and enable accurate and reliable HR detection in various settings.

### SUMMARY

Aspects and advantages of embodiments of the present disclosure will be set forth in part in the following description, or can be learned from the description, or can be learned through practice of the embodiments.

In an aspect, the present disclosure is directed to computer-implemented method for transferring a machine-learned model trained on a first dataset of a first type of radar data to a second dataset of a second type of radar data to leverage learnings from the first and second datasets. The first dataset is larger than the second dataset. The computer-implemented method includes (a) initializing the machine-learned model, (b) training the machine-learned model on the first dataset of the first type of radar data and biometric ground truth data, after (b), then (c) training the machine-learned model on the second dataset of the second type of radar data and the biometric ground truth data, and after (c), then (d) outputting the machine-learned model for use in predicting one or more biometric parameters of a user from the radar data.

In another aspect, the present disclosure is directed to computer-readable media storing a heart rate prediction model. The heart rate prediction model includes an input layer configured to receive input data comprising or derived from radar data, one or more two-dimensional (2-D) ResNet blocks, one or more one-dimensional (1-D) ResNet blocks, and an output layer configured to predict a heart rate.

Other example aspects of the present disclosure are directed to other systems, methods, apparatuses, tangible non-transitory computer-readable media, and devices for performing functions described herein. These and other features, aspects, and advantages of various implementations will become better understood with reference to the following description and appended claims. The accompanying drawings, which are incorporated in and constitute a part of this specification, illustrate implementations of the present disclosure and, together with the description, help explain the related principles.

### BRIEF DESCRIPTION OF THE DRAWINGS

Aspects and advantages of the subject matter will be set forth in part in the following description, or may be obvious from the description, or may be learned through practice of the subject matter.
FIG. 1 depicts a front view of an example mobile computing device according to example embodiments of the present disclosure;
FIG. 2 depicts a rear view of an example mobile computing device according to example embodiments of the present disclosure;
FIG. 3 illustrates a flow chart diagram illustrating a method for transferring a machine-learned model trained on a larger, first dataset of a first type of radar data to a smaller, second dataset of a second type of radar data to leverage learnings from the first and second datasets according to example implementations of aspects of the present disclosure;
FIG. 4 illustrates a block diagram of a processing flow for using machine-learned model(s) to process input(s) to generate output(s) according to example implementations of aspects of the present disclosure;
FIG. 5 illustrates a flow diagram of a high level architecture of a method for training a machine-learned model according to example implementations of aspects of the present disclosure;
FIG. 6 illustrates a flow diagram of an example architecture of model transfer according to example implementations of aspects of the present disclosure;
FIG. 7 illustrates example positions of a mobile computing device configured to collect radar data according to implementations of aspects of the present disclosure;
FIG. 8 illustrates a table of various characteristics of millimeter-wave Frequency-Modulated Continuous Wave (mm-wave FMCW) radar and impulse-radio ultra-wideband (IR-UWB) radar according to example implementations of aspects of the present disclosure;
FIG. 9 illustrates a block diagram of a machine-learned model having one or more two-dimensional (2-D) ResNet and one or more one-dimensional (1-D) ResNet according to example implementations of aspects of the present disclosure;
FIG. 10 illustrates a schematic diagram of flattening mm-wave FMCW radar data performed by an input layer of a machine-learned model according to example implementations of aspects of the present disclosure; and
FIG. 11 illustrates a block diagram of an example networked computing system according to example implementations of aspects of the present disclosure.

These and other features, aspects and advantages of the present subject matter will become better understood with reference to the following description and appended claims. The accompanying drawings, which are incorporated in and constitute a part of this specification, illustrate embodiments of the subject matter and, together with the description, serve to explain the principles of the subject matter.

### DETAILED DESCRIPTION

Reference now will be made in detail to embodiments of the subject matter, one or more examples of which are illustrated in the drawings. Each example is provided by way of explanation of the subject matter, not limitation of the subject matter. In fact, it will be apparent to those skilled in the art that various modifications and variations can be made in the present subject matter without departing from the scope or spirit of the subject matter. For instance, features illustrated or described as part of an embodiment can be used with another embodiment to yield a still further embodiment. Thus, it is intended that the present subject matter covers such modifications and variations as come within the scope of the appended claims and their equivalents.

As used herein, the terms "first," "second," and "third" may be used interchangeably to distinguish one component from another and are not intended to signify location or importance of the individual components. The terms "includes" and "including" are intended to be inclusive in a manner similar to the term "comprising." Similarly, the term "or" is generally intended to be inclusive (e.g., "A or B" is intended to mean "A or B or both"). The term "at least one of" in the context of, e.g., "at least one of A, B, and C" refers to only A, only B, only C, or any combination of A, B, and C. In addition, here and throughout the specification and claims, range limitations may be combined and/or interchanged. Such ranges are identified and include all the sub-ranges contained therein unless context or language indicates otherwise. For example, all ranges disclosed herein are inclusive of the endpoints, and the endpoints are independently combinable with each other. The singular forms "a," "an," and "the" include plural references unless the context clearly dictates otherwise.

Approximating language, as used herein throughout the specification and claims, may be applied to modify any quantitative representation that could permissibly vary without resulting in a change in the basic function to which it is related. Accordingly, a value modified by a term or terms, such as "generally," "about," "approximately," and "substantially," are not to be limited to the precise value specified. In at least some instances, the approximating language may correspond to the precision of an instrument for measuring the value, or the precision of the methods or machines for constructing or manufacturing the components and/or systems. For example, the approximating language may refer to being within a 10 percent margin, i.e., including values within ten percent greater or less than the stated value. In this regard, for example, when used in the context of an angle or direction, such terms include within ten degrees greater or less than the stated angle or direction, e.g., "generally vertical" includes forming an angle of up to ten degrees in any direction, e.g., clockwise or counterclockwise, with the vertical direction V.

The word "exemplary" is used herein to mean "serving as an example, instance, or illustration." In addition, references to "an embodiment" or "one embodiment" do not necessarily refer to the same embodiment, although it may. Any implementation described herein as "exemplary" or "an embodiment" is not necessarily to be construed as preferred or advantageous over other implementations. Moreover, each example is provided by way of explanation of the subject matter, not limitation of the subject matter. In fact, it will be apparent to those skilled in the art that various modifications and variations can be made in the present subject matter without departing from the scope of the subject matter. For instance, features illustrated or described as part of one embodiment can be used with another embodiment to yield a still further embodiment. Thus, it is intended that the present subject matter covers such modifications and variations as come within the scope of the appended claims and their equivalents.

Relative terms such as "below" or "above" or "upper" or "lower" or "horizontal" or "lateral" or "vertical" may be used herein to describe a relationship of one element, layer or region to another element, layer or region as illustrated in the figures. It will be understood that these terms are intended to encompass different orientations of the device in addition to the orientation depicted in the figures. Furthermore, unless otherwise defined, all terms (including technical and scientific terms) used herein have the same meaning as commonly understood by one of ordinary skill in the art to which this subject matter belongs. It will be further understood that terms used herein should be interpreted as having a meaning that is consistent with their meaning in the context of this specification and the relevant art and will not be interpreted in an idealized or overly formal sense unless expressly so defined herein.

In the drawings and specification, there have been disclosed typical embodiments and, although specific terms are employed, they are used in a generic and descriptive sense only and not for purposes of limitation of the scope set forth in the following claims. Furthermore, like numbers refer to like elements throughout. Thus, the same or similar numbers may be described with reference to other drawings even if they are neither mentioned nor described in the corresponding drawing. Also, elements that are not denoted by reference numbers may be described with reference to other drawings.

Generally, the present disclosure is directed to a method of measuring heart rate (HR) using radar technology without needing contact-based sensors, such as those found on wearable devices. More specifically, in an embodiment, the present disclosure relates to a computer-implemented method for transferring a machine-learned model trained on a larger first dataset of a first type of radar data to a second dataset of a second type of radar data to leverage learnings from the first and second datasets. For example, in an embodiment, the present disclosure utilizes two very different types of radar: (1) millimeter-wave Frequency-Modulated Continuous Wave (mm-wave FMCW) and (2) impulse-radio ultra-wideband (IR-UWB) radar for more accurately determining heart rate.

In an embodiment, a large overnight sleep dataset using the mm-wave FMCW radar and simultaneous electrocardiogram (ECG) measurements (the "ground truth" for heart rate) was collected and used to design and train a deep learning model. More specifically, in an embodiment, the deep learning model combined a two-dimensional (2D) ResNet component with a one-dimensional (1D) ResNet component-that processes segments of the radar signal and outputs an estimated heart rate. This model was able to track heart rate with high accuracy and low error (less than 1 beat per minute error on average). Another component of the signal processing includes an "adaptive filter" to remove most of the breathing-related chest movements and isolate the subtler heartbeat signals.

Having proven the approach on mm-wave FMCW radar data, the present disclosure also includes using IR-UWB radar data, which has a more challenging environment, as an input to the deep learning model. For example, IR-UWB radar data has different physical properties (e.g., lower range resolution, different waveforms, fewer antennas). Thus, the present disclosure is directed to "transferring" knowledge from the model trained on abundant mm-wave FMCW data to the new IR-UWB domain. In particular embodiments, the present disclosure further includes adapting pre-processing of the data and then fine-tuning the model with a limited IR-UWB dataset to achieve clinically acceptable levels of heart rate accuracy (e.g., within about four (4) bpm error on average).

Accordingly, the present disclosure illustrates that radar-based heart rate detection can be accurate and convenient. In particular, if a large dataset is used from one radar type, key insights can be transferred to a different radar type with minimal extra data-making it easier to bring contactless heart rate monitoring to new devices. Ultimately, this transfer learning approach helps pave the way for more widespread, continuous, and comfortable heart rate monitoring in everyday life, without needing people to wear or charge additional gadgets.

Various example implementations are described herein with respect to the accompanying Figures.

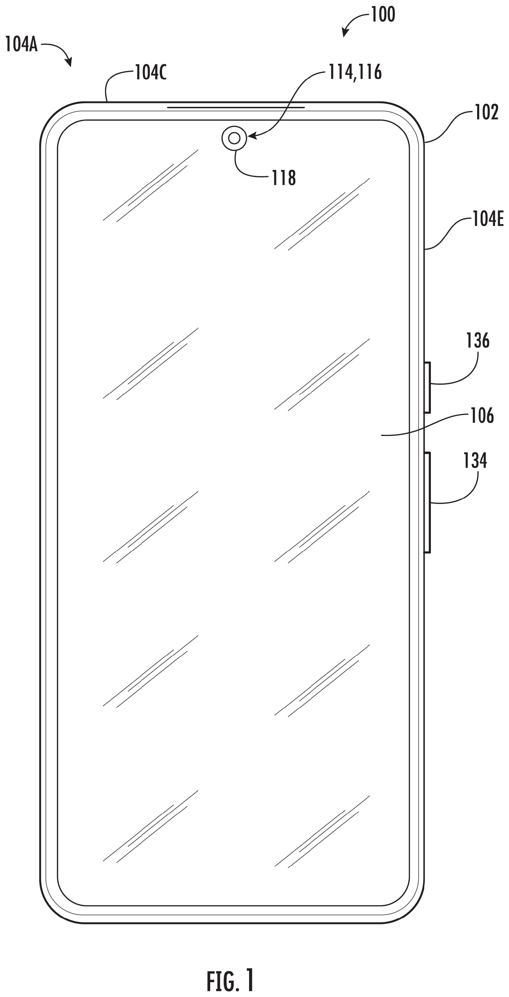

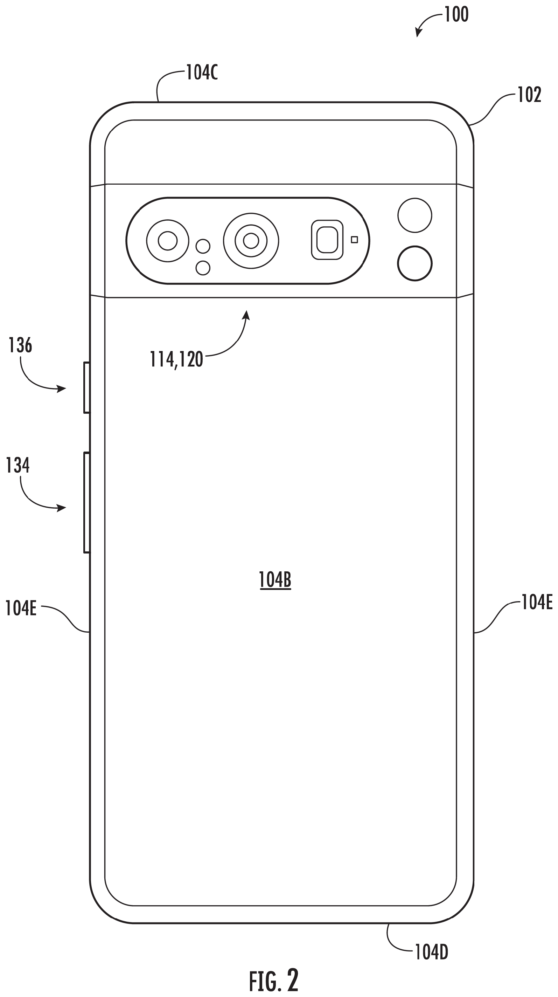

Referring now to the figures, FIGS. 1 and 2 depict an example mobile computing device 100 according to example embodiments of the present disclosure. More particularly, FIG. 1 depicts a front view of the example mobile computing device 100. FIG. 2 depicts a rear view of the example mobile computing device 100. It should be understood that FIGS. 1-2 depict the example mobile computing device 100 and its various components for purposes of illustration and discussion. Those having ordinary skill in the art, using the disclosures provided herein, will appreciate that example aspects of the present disclosure may be implemented by any suitable computing device, such as, by way of non-limiting example, a mobile tablet device, a wearable computing device, and the like.

As generally shown in FIGS. 1 and 2, the mobile computing device 100 may include a housing 102. The housing 102 may include any suitable material, such as aluminum, titanium, and the like. As will be discussed in greater detail below, the housing 102 may define a front surface 104A (e.g., front side), a back surface 104B (e.g., back side), a top surface 104C (e.g., top side), a bottom surface 104D (e.g., bottom side), and one or more side surfaces 104E (e.g., left side, right side, etc.) of the mobile computing device. As shown, the front surface 104A and the back surface 104B of the mobile computing device 100 may be major surfaces/sides, while the top surface 104C, the bottom surface 104D, and the side surface(s) 104E may be minor surfaces/sides. The housing 102 may further define a cavity (e.g., internal volume) (not shown) in which one or more electronic components (e.g., disposed on printed circuit boards) are disposed. For instance, the mobile computing device 100 may include a printed circuit board (e.g., flexible printed circuit board) (not shown) disposed within the cavity defined by the housing 102. The mobile computing device 100 may further include a battery (not shown) that is disposed within the cavity defined by the housing 102. Furthermore, the mobile computing device 100 may also include one or more internal temperature sensors (not shown) within the cavity defined by the housing 102 that are configured to obtain internal temperature data indicative of an internal temperature of the mobile computing device 100.

The mobile computing device 100 may include a display assembly 106. The display assembly 106 may define the front surface 104A (e.g., front side) of the mobile computing device 100. The display assembly 106 may include a display device 108, which may be configured to display content (e.g., time, date, biometric, notifications, etc.) for viewing by the user and to receive inputs from the user. Furthermore, as discussed below, the display assembly 106 may include and/or otherwise be coupled to one or more touch sensors (e.g., touch sensors 112). Hence, the display assembly 106 may be a touch-sensitive display assembly 106 that is sensitive to the touch of a user object (e.g., finger, stylus, and the like). The touch-sensitive display assembly 106 may, in some examples, serve to implement, for instance, a virtual keyboard.

The mobile computing device 100 may also include one or more image capture assemblies 114. For instance, the mobile computing device 100 may include a front image capture assembly 116 on/within the front surface 104A of the mobile computing device 100. The front image capture assembly 116 may include, for instance, a front-facing camera 118 operable to capture images and/or videos. In some examples, the front image capture assembly 116 may be operable to implement a variety of image capture-related tasks, such as autofocus of an aperture and/or lens and the like. It should be understood that the front image capture assembly 116 may include any suitable image capture device without deviating from the scope of the present disclosure. Moreover, as shown in FIG. 2, the mobile computing device 100 may further include a rear image capture assembly 120 on the back surface 104B of the mobile computing device 100.

The mobile computing device 100 may further include one or more buttons and/or ports (hereinafter, input interface elements 130). For instance, in some examples, the mobile computing device 100 may include a power port 132 operable to provide charge to the battery. The mobile computing device 100 may also include one or more volume buttons 134 operable to control a volume of audio output by one or more speakers. The mobile computing device 100 may also include a power button 136 operable to control a power state (e.g., "ON," "OFF," "IDLE," "STANDBY," etc.) of the mobile computing device 100. It should be understood that the mobile computing device 100 may include any suitable button and/or port without deviating from the scope of the present disclosure.

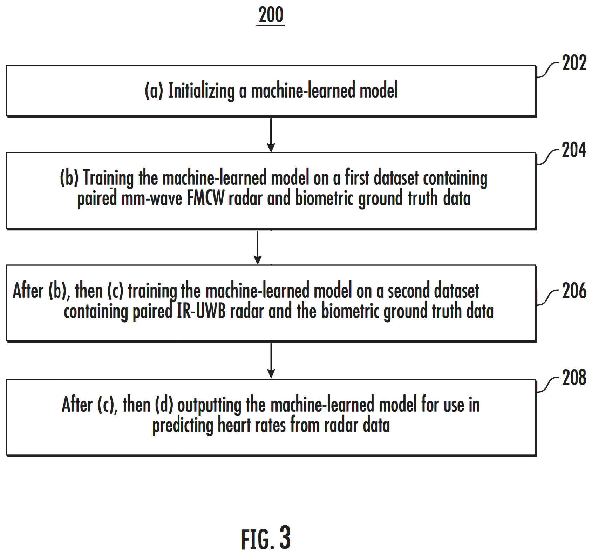

Referring now to FIG. 3, a flow diagram of a method 200 for transferring a machine-learned model trained on a larger, first dataset of a first type of radar data to a smaller, second dataset of a second type of radar data to leverage learnings from the first and second datasets is depicted in accordance with example embodiments of the present disclosure. In certain embodiments, for example, the first dataset of the first type of radar data and the biometric ground truth data may include paired mm-wave Frequency Modulated Continuous Wave (FMCW) radar data and ECG (or PPG) sensor recordings, respectively. Furthermore, in an embodiment, the second dataset of the second type of radar data and the biometric ground truth data may include paired impulse-radio ultra-wideband (IR-UWB) radar data and ECG (or PPG) sensor recordings, respectively. Moreover, it should be appreciated that the example method 200 depicted in FIG. 3 represents merely an example embodiment of the present disclosure, and it should therefore be appreciated that additional and/or alternative steps and procedures may be added to and/or removed from the method 200 without deviating from the scope of the present disclosure.

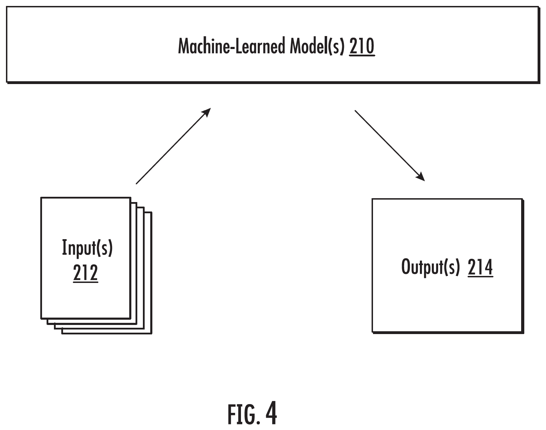

As shown at (202), the method 200 includes (a) initializing a machine-learned model. As an example, FIG. 4 illustrates a block diagram of an example processing flow for using machine-learned model(s) 210 to process input(s) 212 to generate output(s) 214. Machine-learned model(s) 212 can be or include one or multiple machine-learned models or model components. Example machine-learned models can include neural networks (e.g., deep neural networks). Example machine-learned models can include non-linear models or linear models. Example machine-learned models can use other architectures in lieu of or in addition to neural networks. Example machine-learned models can include decision tree based models, support vector machines, hidden Markov models, Bayesian networks, linear regression models, k-means clustering models, etc.

Example neural networks can include feed-forward neural networks, recurrent neural networks (RNNs), including long short-term memory (LSTM) based recurrent neural networks, convolutional neural networks (CNNs), diffusion models, generative-adversarial networks, or other forms of neural networks. Example neural networks can be deep neural networks. Some example machine-learned models can leverage an attention mechanism such as self-attention. For example, some example machine-learned models can include multi-headed self-attention models.

Machine-learned model(s) 212 can include a single or multiple instances of the same model configured to operate on data from input(s) 212. Machine-learned model(s) 212 can include multiple different models or multiple different model portions configured to operate on data from input(s) 212.

Machine-learned model(s) 212 can include an ensemble of different models that can cooperatively interact to process data from input(s) 212. For example, a model ensemble can include multiple models that have different attributes (e.g., different architectures, trained with different recipes, etc.). The ensemble can output an overall output based on the individual outputs of the constituent models. In this manner, for instance, the diverse constituent models can work together to provide system-level robustness by effectively aggregating over individual strengths and weaknesses of any given model. The respective individual outputs can be combined in a weighted combination, using a voting or routing mechanism, or a learned output layer (e.g., one or more feedforward or fully connected layers).

Machine-learned model(s) 212 can employ a mixture-of-experts structure. *See, e.g.,* Zhou et al., Mixture-of-Experts with Expert Choice Routing, ARX1v:2122120212.0921468v212 (Oct. 212104, 2120212212). For example, different portions of a model can learn (explicitly or implicitly) different expertise areas, with pathways through the model being selected by a learned routing mechanism that engages the appropriate expert for a given input (e.g., a given portion of an input, such as on a per-token basis). For example, a feedforward network can be sparsely activated for a given portion of an input based on an output of a routing mechanism that processes the portion of the input. In this manner, for instance, the group of activated weights can form an "expert" that is selected by the router. On each forward pass, only a subset of the total model weights may be engaged, thereby decreasing a quantity of operations performed for processing a given input compared to a densely activated model. In this manner, for instance, the expressive and interpretive power of a high-parameter-count model can be achieved with more compute-efficient forward passes.

Still referring to FIG. 4, the input(s) 212 can generally include or otherwise represent various types of data. Input(s) 212 can include one type or many different types of data. The output(s) 214 can be data of the same type(s) or of different types of data as compared to input(s) 212. Further, the output(s) 214 can include one type or many different types of data.

Example data types for input(s) 212 or output(s) 214 include natural language text data, software code data (e.g., source code, object code, machine code, or any other form of computer-readable instructions or programming languages), machine code data (e.g., binary code, assembly code, or other forms of machine-readable instructions that can be executed directly by a computer's central processing unit), assembly code data (e.g., low-level programming languages that use symbolic representations of machine code instructions to program a processing unit), genetic data or other chemical or biochemical data, image data, audio data, audiovisual data, haptic data, biometric data, medical data, financial data, statistical data, geographical data, astronomical data, historical data, sensor data generally (e.g., digital or analog values, such as voltage or other absolute or relative level measurement values from a real or artificial input, such as from an audio sensor, light sensor, displacement sensor, etc.), and the like. Data can be raw or processed and can be in any format or schema.

In multimodal inputs 212 or outputs 214, example combinations of data types include image data and audio data, image data and natural language data, natural language data and software code data, image data and biometric data, sensor data and medical data, etc. It is to be understood that any combination of data types in an input 212 or an output 214 can be present.

An example input 212 can include one or multiple data types, such as the example data types noted above. An example output 214 can include one or multiple data types, such as the example data types noted above. The data type(s) of input 212 can be the same as or different from the data type(s) of output 214. It is to be understood that the example data types noted above are provided for illustrative purposes only. Data types contemplated within the scope of the present disclosure are not limited to those examples noted above.

Referring back to FIG. 3, as shown at (204), the method 200 includes (b) training the machine-learned model on the first dataset of the first type of radar data and biometric ground truth data. As shown at (206), the method 200 includes, after (b), then (c) training the machine-learned model on the second dataset of the second type of radar data and the biometric ground truth data. For example, in an embodiment, training the machine-learned model on the second dataset of the second type of radar data and the biometric ground truth data may include fine-tuning the machine-learned model. In such embodiments, fine-tuning can include, for instance, smaller-scale training on higher-quality (e.g., labeled, curated, etc.) data. As such, fine-tuning can affect all or a portion of the parameters of the machine-learned model. For example, various portions of the machine-learned model can be "frozen" for certain training stages. Further, as an example, parameters associated with an embedding space can be "frozen" during fine-tuning (e.g., to retain information learned from a broader domain(s) than present in the fine-tuning dataset(s)). During the fine-tuning process, the original parameters of the pre-trained model are typically frozen, and only the parameters of the adapters are updated.

In some implementations, the method 200 can be implemented to execute parameter-efficient fine-tuning methods, such as Low-Rank Adaptation (LoRA). LoRA can refine pre-trained models with minimal adjustments to the original parameters. This can be achieved by introducing trainable low-rank matrices that modify the behavior of the pre-trained weights without directly altering them. In some implementations, during fine-tuning, only these auxiliary matrices are updated, which significantly reduces the number of parameters that are trained.

Referring still to FIG. 3, as shown at (208), the method 200 includes after (c), then (d) outputting the machine-learned model for use in predicting one or more biometric parameters of a user from the radar data.

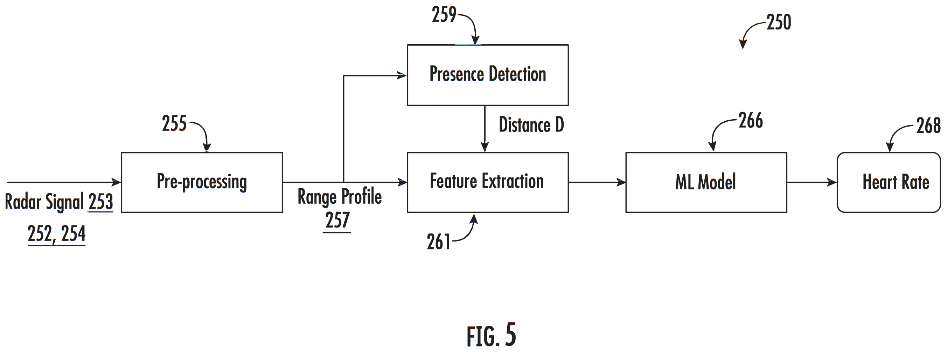

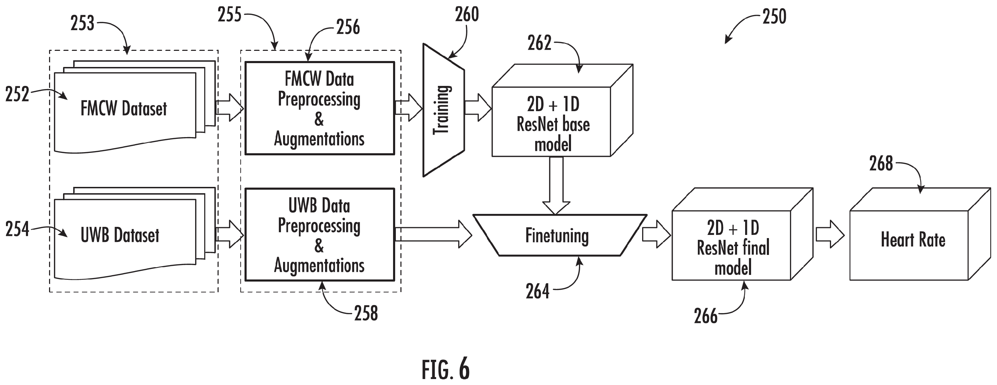

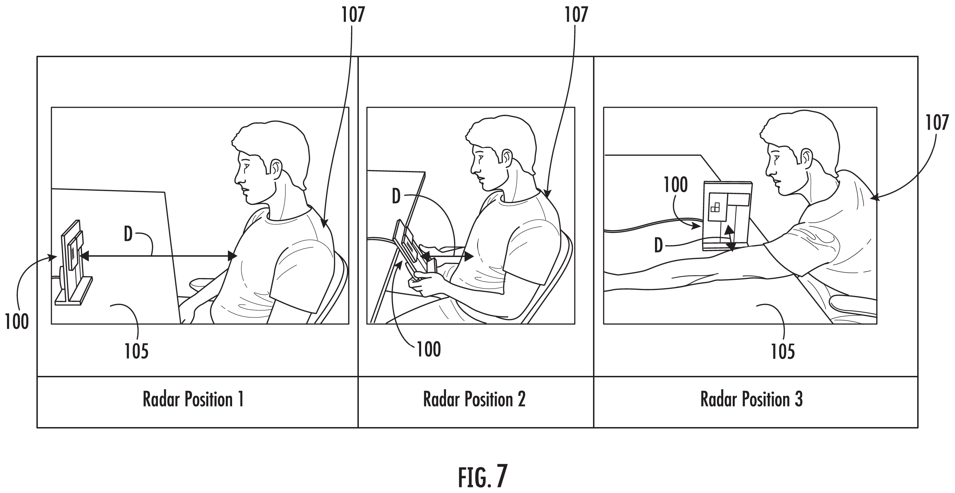

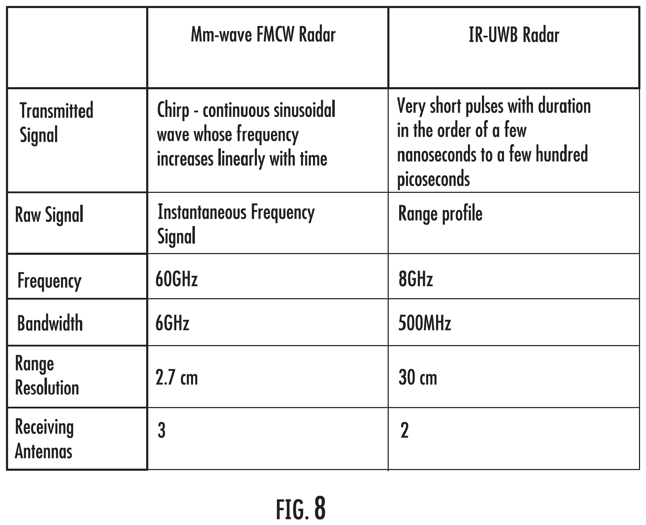

The method of FIG. 3 can be better understood with respect to FIGS. 5-8. More particularly, FIG. 5 illustrates a flow diagram of an example architecture 250 of model transfer according to example implementations of aspects of the present disclosure; FIG. 6 illustrates a flow diagram of an example architecture 250 of model transfer according to example implementations of aspects of the present disclosure; FIG. 7 illustrates example positions of a mobile computing device configured to collect radar data according to implementations of aspects of the present disclosure; and FIG. 8 illustrates a table of various characteristics of millimeter-wave Frequency-Modulated Continuous Wave (mm-wave FMCW) radar and impulse-radio ultra-wideband (IR-UWB) radar according to example implementations of aspects of the present disclosure.

Referring particularly to FIGS. 5 and 6, example diagrams of an architecture 250 for training a machine-learned model 266 according to example implementations of aspects of the present disclosure are illustrated. More specifically, as shown, the architecture 250 receives radar signal(s) 253 that include a mm-wave FMCW radar dataset 252 and an IR-UWB radar dataset 254. The radar datasets 252, 254 may be collected using, e.g., one or more mobile computing devices. For example, the mobile computing device(s) may be the mobile computing device 100 of FIGS. 1 and 2, which corresponds to a smartphone, or may also include one or more of a smart display, a tablet, a laptop, a desktop, or a wearable computing device. For example, in an embodiment, the mm-wave FMCW radar dataset 252 may be collected using a smart display. Similarly, the IR-UWB radar dataset 254 may be collected using any suitable mobile computing device in any suitable position, such as the positions illustrated in FIG. 7. More specifically, as shown in FIG. 7, radar position 1 corresponds to spacing the mobile computing device 100 on a fixed surface 105 at a distance from a user 107; radar position 2 corresponds to the user 107 holding the mobile computing device 100 at a distance away; and radar position 3 corresponds to the user 107 placing a limb (or any other suitable body part) in proximity to the mobile computing device 100, which is resting on the fixed surface 105.

Moreover, and referring back to FIGS. 5 and 6, once collected, each dataset 252, 254 is subsequently pre-processed 255 and/or augmented as shown at 256 and 258, respectively. For example, as shown in Table 1 of FIG. 8, mm-wave FMCW and IR-UWB radar datasets have very different characteristics. As an example, FMCW radar has one transmitting and three receiving antennas, and a bandwidth of 5.5 GHz which leads to the range resolution of 0.027 m. In contrast, in an example, the IR-UWB radar has one transmitting and two receiving antennas, and a bandwidth of 0.5 GHz, which leads to about a 10 times lower range resolution of 0.3 m. Thus, in an embodiment, to achieve a transferable model from the mm-wave FMCW radar to the IR-UWB radar, the present disclosure performed the additional preprocessing steps, as shown at 256 and 258, to modify the mm-wave FMCW radar dataset to better resemble the target IR-UWB data.

The additional preprocessing steps aim to address various issues, such as the different number of receiving antennas and the different range resolution. To address the different number of receiving antennas, the additional preprocessing steps may include concatenate all antennas into a single example when training on the FMCW radar dataset 252 and finetuning on the IR-UWB radar dataset 254. This process results in a different size of the input data cube between those two datasets. Still another preprocessing step may include splitting each antenna into a separate example, training, and then finetuning the machine-learned model on all available antennas. Still further pre-processing steps may include choosing a single antenna when training and fine-tuning, selecting a pair of two antennas when training and fine-tuning on all two available antennas, performing a Principal Component Analysis (PCA) beamforming prior to feature extraction step, then training and fine-tuning on features extracted from one beamformed antenna, and/or performing a convolution step to combine features from three antennas during training, and from two antennas during finetuning.

To address the different range resolution, the additional preprocessing steps may include selecting two times the n number of range bins when training the machine-learned model and n number of range bins when fine-tuning the model, which results in a different size of the input data cube between those two datasets. Still another preprocessing step may include down-sampling the range bins of the FMCW radar dataset 252 by averaging, e.g., 10 range bins into a single range bin when training the machine-learned model and/or down-sampling, e.g., 10 range bins to one by applying a gaussian kernel when training the machine-learned model. Still further pre-processing steps may include selecting n number of range bins when training the machine-learned model and n number of range bins when finetuning the machine-learned model, which results in a different physical area being considered on each dataset. In still further embodiments, certain augmentation techniques may be applied to the FMCW radar dataset 252 and/or the IR-UWB radar dataset 254 when training the machine-learned model, such as applying the gaussian noise to the input data and swapping the order of features (magnitude and unwrapped angle) for each range bin / antenna.

Thus, in an embodiment, at a pre-processing step for the FMCW radar dataset 252, the chirps within each burst may be averaged, resulting in a raw FMCW radar signal with 30 Hz sampling rate and 256 samples per chirp. Then, this data can be split into overlapping segments, thereby resulting in a data cube having certain dimensions relating to the number of samples per chirp (also referred to as "fast-time" axis), the number of receiving antennas, and the number of bursts within a certain time frame segment (also referred to as "slow-time" axis). Further, in an embodiment, for each segment, a clutter filter may be applied to eliminate stationary background clutter. In such embodiments, the clutter filter is performed by first calculating the average signal along the "slow-time" axis and then subtracting it from the original signal. The pre-processing step can then include computing the fast Fourier transform along the "fast-time" axis, which results in the complex-valued range profile, having a certain dimension relating to the number of range bins, the number of receiving antennas, and the "slow-time" axis.

Moreover, in an embodiment, at a pre-processing step for the IR-UWB dataset 254, the raw IR-UWB signal is already represented as a range profile. Thus, during the preprocessing step, the signal can be downsampled, e.g., from 200 Hz to 30 Hz to match the FMCW radar's sample rate by averaging the samples in the downsample window. Similar to the FMCW radar dataset 252, the data may be split into overlapping segments, with a certain step size, resulting in a data cube with a certain dimension as a function of the number of range bins, the number of receiving antennas, and the number of timesteps within that segment.

Referring particularly to FIG. 5, the corresponding range profile 257 may be input into a presence detection module 259 and a feature extraction module 261. In an embodiment, the purpose of the presence detection module 259 is to detect the user and its distance D from the radar (i.e., the mobile computing device 100), e.g., as shown in FIG. 7. In such embodiments, the same algorithm can be used for both the FMCW radar dataset 252 and the IR-UWB dataset 254. Given the complex-valued range profile, the architecture 250 may first perform a clutter filter by calculating the average along the "slow-time" axis and subtracting it from the original signal. In particular embodiments, the architecture 250 can then use the Constant false alarm rate (CFAR) algorithm, e.g., with a threshold of 1.5 to detect the range bin index with the highest power. The resulting range bin index is considered to be a user's location. If the user cannot be detected, i.e., there is no range bin with the peak exceeding the threshold, the segment is rejected.

Heart rate can be estimated from a radar signal by measuring the movements of the target's chest or other parts of the body caused by heart beats. Chest movements caused by heart beats are very subtle, e.g., having an amplitude of 0.2-0.5 mm. The heart beats can also be detected from other parts of the body by measuring the displacement of the skin, whose amplitude is in tens of micrometers. Accordingly, given the complex-number range profile and the range bin index where the user was detected, a number of range bins around the user can be extracted using the feature extraction module 261.

For example, due to the small amplitude and low frequency of the FMCW radar dataset 252, the signals in this dataset are sufficient to measure the phase change between successive chirps in "slow-time". Hence typically, only the unwrapped phase from the complex-valued range profile is extracted. The phase of the signal is calculated using an arctangent function with the value between [-Pi, Pi]. In certain embodiments, the unwrap operation is needed to reconstruct the signal's original phase by removing the 2Pi jumps in the signal.

In certain embodiments, while the unwrapped angle of the range profile is useful to estimate HR from the mm-wave radar signal, a significant boost in performance may be achieved if the magnitude of the signal is included as well. Hence, in an embodiment, for each range bins selected, the magnitude and unwrapped angle can be extracted by the feature extraction module 261 as two separate signals.

On the other hand, since IR-UWB radar uses the pulse to measure range, the body movements caused by heart rate can be measured by the amplitude. Hence typically, only the magnitude component of the complex-valued range profile is extracted. However, in the present disclosure, the feature extraction module 261 provides a boost in performance by extracting the magnitude and unwrapped angle when estimating HR from the IR-UWB dataset 254. In further embodiments, each signal may then also be filtered using, e.g., a high pass filter to eliminate the low frequency noise, e.g., below 3 Hz.

Historically, a challenge in estimating HR from radar is that not only are the movements caused by heart beats very small, but they are also often shadowed by the breathing motion and its harmonics, other body movements, and environmental noise. Thus, to reliably extract the HR frequency from radar signals, pre-processing techniques are usually employed. Prior work suggests using band-pass filters to only include the range of frequencies of interest, however this only filters out the dominant frequency of the respiratory rate (RR), leaving its harmonics that can still shadow the HR frequency. Moreover, during sleep, while the normal resting HR is typically between 60 - 100 beats-per-minute (bpm), it can drop to as low as 40 bpm. Moreover, while the normal range of the RR is 12 - 20 bpm, it could be as high as 44 bpm, which overlaps with the HR range. Therefore, it can be difficult to define a cutoff frequency, which would effectively filter out the RR across the dataset, while not impacting the HR.

To address these issues, the architecture 250 described herein utilizes an adaptive filter to effectively filter the RR and its first harmonic and is derived adaptively per sample. For example, in an embodiment, the RR trend can be derived from the signal by using, e.g., a 3rd order Savitzky-Golay filter with a filter window length of, e.g., 1.5 seconds. In an embodiment, this trend can be subtracted from the original signal, which removes the RR's dominant frequency and its 1st harmonic from the signal. The resulting signal contains the HR frequency which is much easier to extract. Furthermore, in certain embodiments, the adaptive filter can be applied on both the magnitude and the unwrapped angle of each extracted range bin from the range profile from each receiving antenna.

Referring back to FIG. 6, as shown at 260 and 262, the example architecture 250 further includes initially training the machine-learned model on the preprocessed data (e.g., the mm-wave FMCW radar dataset 252). Further, as shown at 264, the example architecture 250 includes further training (e.g., fine-tuning) the machine-learned model on the IR-UWB radar dataset 254. As shown at 266, the final trained, machine-learned model is output for use in predicting one or more biometric parameters, such as heart rate 268 of a user.

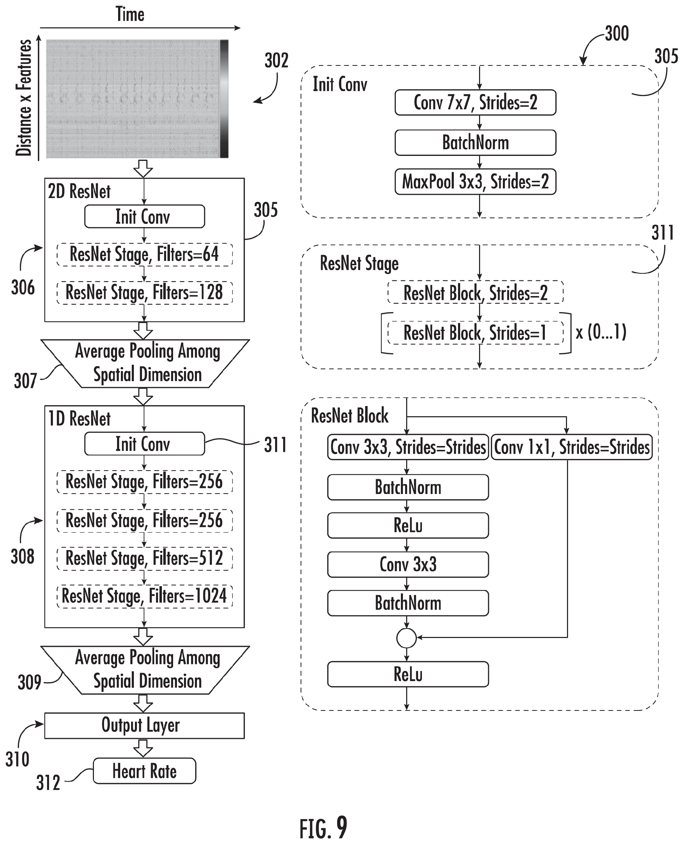

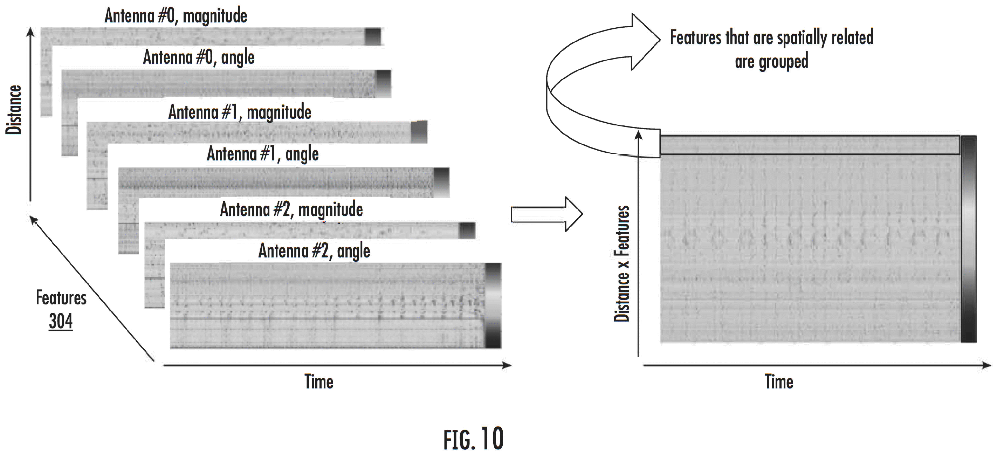

Referring now to FIG. 9, a block diagram of a machine-learned model 300, such as a heart rate prediction model corresponding to the final trained, machine-learned model 266 described herein, having one or more two-dimensional (2-D) ResNet and one or more one-dimensional (1-D) ResNet according to example implementations of aspects of the present disclosure is illustrated. More specifically, as shown, the machine-learned model 300 includes an input layer 302 configured to receive input data including or derived from radar data. In an embodiment, the input data may be IR-UWB radar data as described herein. Moreover, as an example, FIG. 10 illustrates a schematic diagram of flattened mm-wave FMCW radar data that may be used to train the heart rate prediction model according to example implementations of aspects of the present disclosure (e.g., the flattened mm-wave FMCW radar data may include only one antenna and only one range bin). In particular, as shown in FIG. 10, the radar data may include extracted features 304 from the flattened mm-wave FMCW radar data that are reshaped to an input of T x S x C, where T is a time dimension, S is a spatial dimension having at least two features, and C is a number of channels. In such embodiments, the spatial dimension is a function of a number of antennas and a number of range bins. In other words, the features 304 that are spatially related can be grouped together. For example, as shown, the features 304 may include a magnitude and/or an unwrapped angle per each of the number of antennas per each of the number of range bins, flattened, to ensure spatially related features are grouped together. In an embodiment, the features 304 may also be scaled, e.g., using minimum-maximum normalization.

Referring back to FIG. 9, the machine-learned model 300 further includes one or more two-dimensional (2-D) ResNet blocks 306 and one or more one-dimensional (1-D) ResNet blocks 308. As used herein, 2-D and 1-D ResNets are variants of ResNet architectures adapted for processing 2-D (e.g., images) and 1-D (e.g., sequences) data, respectively. Both 2-D and 1-D ResNets utilize residual connections to enable deeper networks but differ in how they perform convolutions. For example, 2-D ResNets use 2-D convolutions on 2-D input, while 1-D ResNets use 1-D convolutions on 1-D input.

More specifically, as shown, the machine-learned model 300 includes the 2-D ResNet block(s) 306 followed by the 1-D ResNet block(s) 308. In such embodiments, a rationale behind using the 2-D ResNet block(s) 306 at the first stage is that both spatial and temporal relationships between features can be useful in the early layers since features that are spatially close reflect the vibrations from neighboring parts of the body. Further, as shown, the 2-D ResNet block(s) 306 may contain two stages, with 64 and 128 filters, respectively. Moreover, as shown at 305, the 2-D ResNet block(s) 306 may use a stride of 2, in order to increase receptive fields and enable feature hierarchy. Each stage may include up to two ResNet blocks. Thus, in the illustrated example, an output of the 2-D ResNet block(s) 306 is a 3-D data cube of size T' x S' x 128, where T' and S' are the temporal and spatial dimensions respectively, which are smaller than the original input size due to striding and polling, and 128 is the number of features.

As shown at 307, the machine-learned model 300 may further include averaging features of the 3-D data cube among the spatial dimension, thereby resulting in a 2-D data cube, e.g., of a size of T' x 128. The resulting data cube can then be fed into the 1-D ResNet block(s) 308 to extract one or more specific features among the time dimension in the increasingly larger receptive field. As an example, the 1-D ResNet block(s) 308 includes four stages, with 128, 256, 512 and 1024 filters, respectively. Similar to above, as shown at 311, the 1-D ResNet block(s) 308 may use a stride of 2. The resulting data cube has size T'' x 1024, where T" is a temporal dimension which is smaller than T' due to striding and pooling and 1024 is the number of features. At the end, the features for all timesteps are averages, resulting in 1-D data of size 1024. In addition, as shown, the machine-learned model 300 includes an output layer 310 configured to predict a heart rate 312. In such embodiments, the output layer 310 may correspond to a fully connected layer used to predict the heart rate 312 from the extracted feature(s).

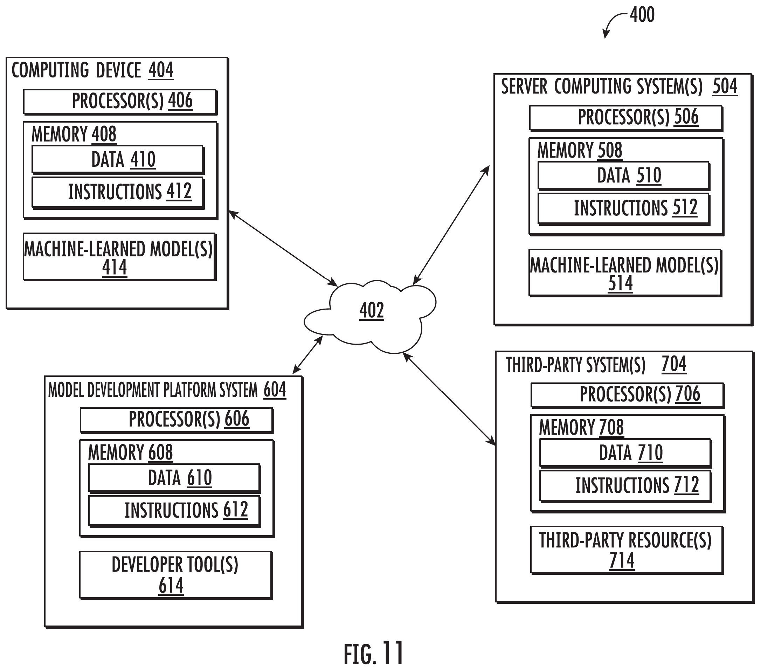

Referring now to FIG. 11, a block diagram of an example networked computing system 400 that can perform aspects of example implementations of the present disclosure. The computing system 400 can include a number of computing devices and systems that are communicatively coupled over a network 402. An example computing device 404 is described to provide an example of a computing device that can perform any aspect of the present disclosure. An example server computing system 504 is described as an example of a server computing system that can perform any aspect of the present disclosure. The computing device 404 and the server computing system(s) 504 can cooperatively interact (e.g., over the network 402) to perform any aspect of the present disclosure. A model development platform system 604 is an example system that can host or serve a model development platform(s) for development of machine-learned models. Third-party system(s) 704 are example system(s) with which any of computing device 404, the server computing system(s) 504, or model development platform system(s) 604 can interact in the performance of various aspects of the present disclosure (e.g., engaging third-party tools, accessing third-party databases or other resources, etc.).

The network 402 can be any type of communications network, such as a local area network (e.g., intranet), wide area network (e.g., Internet), or some combination thereof and can include any number of wired or wireless links. In general, communication over network 402 can be carried via any type of wired or wireless connection, using a wide variety of communication protocols (e.g., TCP/IP, HTTP, SMTP, FTP), encodings or formats (e.g., HTML, XML), or protection schemes (e.g., VPN, secure HTTP, SSL). The network 402 can also be implemented via a system bus.

The computing device 404 can be any type of computing device, such as, for example, a personal computing device (e.g., laptop or desktop), a mobile computing device (e.g., smartphone or tablet), a gaming console or controller, a wearable computing device, an embedded computing device, a server computing device, a virtual machine operating on a host device, or any other type of computing device. The computing device 404 can be a client computing device. The computing device 404 can be an end-user computing device. The computing device 404 can be a computing device of a service provided that provides a service to an end user (who may use another computing device to interact with the computing device 404).

The computing device 404 can include one or more processors 406 and a memory 408. The processor(s) 406 can be any suitable processing device (e.g., a processor core, a microprocessor, an ASIC, an FPGA, a controller, a microcontroller, etc.) and can be one processor or a plurality of processors that are operatively connected. The memory 408 can include one or more non-transitory computer-readable storage media, such as HBM, RAM, ROM, EEPROM, EPROM, flash memory devices, magnetic disks, etc., and combinations thereof. The memory 408 can store data 410 and instructions 412 which can be executed by processor(s) 406 to cause computing device 404 to perform operations. The operations can implement any one or multiple features described herein. The operations can implement example methods and techniques described herein.

The computing device 404 can also include one or more input components that receive user input. For example, a user input component can be a touch-sensitive component (e.g., a touch-sensitive display screen or a touch pad) that is sensitive to the touch of a user input object (e.g., a finger or a stylus). The touch-sensitive component can serve to implement a virtual keyboard. Other example user input components include a microphone, camera, LIDAR, a physical keyboard or other buttons, or other means by which a user can provide user input.

The computing device 404 can store or include one or more machine-learned models 414. The machine-learned models 414 can include, e.g., the machine-learned model2 210, 266, 300 described herein. The machine-learned models 414 can be received from the server computing system(s) 504, model development platform system 604, the third party system(s) 704 (e.g., an application distribution platform), or developed locally on the computing device 404. The machine-learned model(s) 414 can be loaded into memory 408 and used or otherwise implemented by processor(s) 406. The computing device 404 can implement multiple parallel instances of machine-learned model(s) 414.

The server computing system(s) 504 can include one or more processors 506 and a memory 508. The processor(s) 506 can be any suitable processing device (e.g., a processor core, a microprocessor, an ASIC, an FPGA, a controller, a microcontroller, etc.) and can be one processor or a plurality of processors that are operatively connected. The memory 508 can include one or more non-transitory computer-readable storage media, such as HBM, RAM, ROM, EEPROM, EPROM, flash memory devices, magnetic disks, etc., and combinations thereof. The memory 508 can store data 510 and instructions 512 which can be executed by processor(s) 506 to cause server computing system(s) 504 to perform operations. The operations can implement any one or multiple features described herein. The operations can implement example methods and techniques described herein.

In some implementations, the server computing system 504 includes or is otherwise implemented by one or multiple server computing devices. In instances in which server computing system 504 includes multiple server computing devices, such server computing devices can operate according to sequential computing architectures, parallel computing architectures, or some combination thereof.

The server computing system 504 can store or otherwise include one or more machine-learned models 514. The machine-learned model(s) 514 can be the same as or different from machine-learned model(s) 414. The machine-learned model(s) 514 can be received from the computing device 404, the model development platform system 604, the third party system(s) 704, or developed locally on server computing system(s) 504. The machine-learned model(s) 514 can be loaded into memory 508 and used or otherwise implemented by processor(s) 506. The server computing system(s) 504 can implement multiple parallel instances of machine-learned model(s) 514.

In an example configuration, the machine-learned models 514 can be included in or otherwise stored and implemented by the server computing system 504 to establish a client-server relationship with the computing device 404 for serving model inferences. For instance, the machine-learned models 514 can be implemented by the server computing system 504 as a portion of a web service (e.g., remote machine-learned model hosting service, such as an online interface for performing machine-learned model operations over a network on server computing system(s) 504). For instance, the server computing system(s) 504 can communicate with the computing device 404 over a local intranet or internet connection. For instance, the computing device 404 can be a workstation or endpoint in communication with server computing system(s) 504, with implementation of machine-learned models 514 being managed by server computing system(s) 504 to remotely perform inference (e.g., for runtime or training operations), with output(s) returned (e.g., cast, streamed, etc.) to computing device 404. The machine-learned models 514 can work cooperatively or interoperatively with the machine-learned models 414 on the computing device 404 to perform various tasks.

The model development platform system(s) 604 can include one or more processors 606 and a memory 608. The processor(s) 606 can be any suitable processing device (e.g., a processor core, a microprocessor, an ASIC, an FPGA, a controller, a microcontroller, etc.) and can be one processor or a plurality of processors that are operatively connected. The memory 608 can include one or more non-transitory computer-readable storage media, such as HBM, RAM, ROM, EEPROM, EPROM, flash memory devices, magnetic disks, etc., and combinations thereof. The memory 608 can store data 610 and instructions 612 which can be executed by processor(s) 606 to cause model development platform system(s) 604 to perform operations. The operations can implement any one or multiple features described herein. The operations can implement example methods and techniques described herein. This and other functionality can be implemented by developer tool(s) 614.

The third-party system(s) 704 can include one or more processors 706 and a memory 708. The processor(s) 706 can be any suitable processing device (e.g., a processor core, a microprocessor, an ASIC, an FPGA, a controller, a microcontroller, etc.) and can be one processor or a plurality of processors that are operatively connected. The memory 708 can include one or more non-transitory computer-readable storage media, such as HBM, RAM, ROM, EEPROM, EPROM, flash memory devices, magnetic disks, etc., and combinations thereof. The memory 708 can store data 710 and instructions 712 which can be executed by processor(s) 706 to cause third-party system(s) 704 to perform operations. The operations can implement any one or multiple features described herein. The operations can implement example methods and techniques described herein. Example operations include the functionality described herein with respect to tools and other external resources called when training or performing inference with machine-learned model(s).

The technology discussed herein makes reference to servers, databases, software applications, and other computer-based systems, as well as actions taken and information sent to and from such systems. The inherent flexibility of computer-based systems allows for a great variety of possible configurations, combinations, and divisions of tasks and functionality between and among components. For instance, processes discussed herein can be implemented using a single device or component or multiple devices or components working in combination. Databases and applications can be implemented on a single system or distributed across multiple systems. Distributed components can operate sequentially or in parallel.

While the present subject matter has been described in detail with respect to various specific example embodiments thereof, each example is provided by way of explanation, not limitation of the disclosure. Those skilled in the art, upon attaining an understanding of the foregoing, can readily produce alterations to, variations of, and equivalents to such embodiments. Accordingly, the subject disclosure does not preclude inclusion of such modifications, variations or additions to the present subject matter as would be readily apparent to one of ordinary skill in the art. For instance, features illustrated or described as part of one embodiment can be used with another embodiment to yield a still further embodiment. Thus, it is intended that the present disclosure cover such alterations, variations, and equivalents.

The proposed solutions in particular relates to the following embodiments:
Embodiment 1:
   A computer-implemented method for transferring a machine-learned model trained on a first dataset of a first type of radar data to a second dataset of a second type of radar data to leverage learnings from the first and second datasets, the first dataset being larger than the second dataset, the computer-implemented method comprising:
   (a) initializing the machine-learned model;
   (b) training the machine-learned model on the first dataset of the first type of radar data and biometric ground truth data;

   after (b), then (c) training the machine-learned model on the second dataset of the second type of radar data and the biometric ground truth data; and
   after (c), then (d) outputting the machine-learned model for use in predicting one or more biometric parameters of a user from the radar data.
Embodiment 2: The computer-implemented method of Embodiment 1, wherein the first dataset of the first type of radar data comprises paired mm-wave Frequency Modulated Continuous Wave (FMCW) radar data.
Embodiment 3: The computer-implemented method of Embodiment 1 or 2, wherein the second dataset of the second type of radar data comprises paired impulse-radio ultra-wideband (IR-UWB) radar data.
Embodiment 4: The computer-implemented method of any one of Embodiments 1 to 3, wherein the biometric ground truth data comprises at least one of electrocardiogram (ECG) signals or photoplethysmogram (PPG) signals.
Embodiment 5: The computer-implemented method of any one of Embodiments 1 to 4, further comprising collecting at least one of the first dataset of the first type of radar data or the second dataset of the second type of radar data using one or more mobile computing devices. Embodiment 6: The computer-implemented method of Embodiment 5, wherein the one or more mobile computing devices comprises at least one of a smartphone, a smart display, a tablet, a laptop, a desktop, or a wearable computing device.
Embodiment 7: The computer-implemented method of any one of Embodiments 1 to 6, wherein the machine-learned model comprises at least one of one or more two-dimensional ResNet blocks or one or more one-dimensional ResNet blocks.
Embodiment 8: The computer-implemented method of Embodiment 7, wherein the machine-learned model comprises the one or more two-dimensional ResNet blocks followed by the one or more one-dimensional ResNet blocks.
Embodiment 9: The computer-implemented method of any one of Embodiments 1 to 8, wherein the one or more biometric parameters of the user comprises a heart rate signal. Embodiment 10: The computer-implemented method of any one of Embodiments 1 to 9, further comprising pre-processing at least one of the first dataset of the first type of radar data or the second dataset of the second type of radar data before training the machine-learned model.
Embodiment 11: The computer-implemented method of Embodiment 10, wherein pre-processing at least one of the first dataset of the first type of radar data or the second dataset of the second type of radar data further comprises applying one or more filters derived adaptively from a respiratory rate per sample to at least one of the first dataset of the first type of radar data or the second dataset of the second type of radar data to facilitate extraction of the heart rate signal.
Embodiment 12: The computer-implemented method of Embodiment 11, wherein applying the one or more filters further comprises applying the one or more filters to a magnitude and an unwrapped angle of each extracted range bin from a range profile from each receiving antenna used to collect at least one of the first dataset of the first type of radar data or the second dataset of the second type of radar data.
Embodiment 13: Computer-readable media storing instructions which, when executed by at least one processor of a heart monitoring systems, cause the heart monitoring system to carry out a method of any one of Embodiments 1 to 12.
Embodiment 14: A heart monitoring system, comprising at least one processor and a memory, the memory including instructions, which, when executed by the at least one processor, cause the heart monitoring system to carry out a method of any one of Embodiments 1 to 12. Embodiment 15: The heart monitoring system of Embodiment 14, comprising one or more mobile computing devices for collecting least one of the first dataset of the first type of radar data or the second dataset of the second type of radar data.
Embodiment 16:
   Computer-readable media storing a heart rate prediction model, the heart rate prediction model comprising:
   an input layer configured to receive input data comprising or derived from radar data;
   one or more two-dimensional (2-D) ResNet blocks;
   one or more one-dimensional (1-D) ResNet blocks; and
   an output layer configured to predict a heart rate.
Embodiment 17: The computer-readable media of claim 16, wherein the radar data comprises flattened mm-wave Frequency Modulated Continuous Wave (FMCW) radar data used to train the heart rate prediction model.
Embodiment 18. The computer-readable media of Embodiment 16 or 17, wherein the input data comprises impulse-radio ultra-wideband (IR-UWB) radar data.
Embodiment 19: The computer-readable media of any one of Embodiments 16 to 8, wherein the input layer comprises extracted features from the radar data that are reshaped to an input of T x S x C, where T is a time dimension, S is a spatial dimension having at least two features, and C is a number of channels.
Embodiment 20: The computer-readable media of Embodiment 19, wherein the spatial dimension is a function of a number of antennas and a number of range bins.
Embodiment 21: The computer-readable media of Embodiment 20, wherein the at least two features comprises a magnitude and an unwrapped angle per each of the number of antennas per each of the number of range bins, flattened, to ensure spatially related features are grouped together.
Embodiment 22: The computer-readable media of Embodiment 21, wherein the at least two features are scaled using minimum-maximum normalization.
Embodiment 23: The computer-readable media of any one of Embodiments 16 to 22, wherein the one or more 2-D ResNet blocks are followed by the one or more 1-D ResNet blocks. Embodiment 24: The computer-readable media of Embodiment 23, wherein a first 2-D ResNet block of the one or more 2-D ResNet blocks generates a three-dimensional (3-D) data cube having smaller time and spatial dimensions than the input.
Embodiment 25: The computer-readable media of Embodiment 24, wherein features of the 3-D data cube are averaged among the spatial dimension.
Embodiment 26: The computer-readable media of Embodiment 24 or 25, wherein the 3-D data cube is fed into a first 1-D ResNet block of the one or more 1-D ResNet blocks to extract one or more specific features among the time dimension.
Embodiment 27: The computer-readable media of any one of Embodiments 16 to 26, wherein the output layer is a fully connected layer used to predict the heart rate from the one or more specific features.
Embodiment 28: The computer-readable media of any one of Embodiments 16 to 27, wherein the radar data is collected from one or more mobile computing devices.
Embodiment 29: The computer-readable media of Embodiment 28, wherein the one or more mobile computing devices comprises at least one of a smartphone, a smart display, a tablet, a laptop, a desktop, or a wearable computing device.
Embodiment 30: The computer-readable media of any one of Embodiments 16 to 29, wherein the radar data is pre-processed.
Embodiment 31: The computer-readable media of Embodiment 30, wherein the radar data is pre-processed using one or more filters derived adaptively from a respiratory rate per sample to facilitate extraction of the heart rate.
Embodiment 32: The computer-readable media of Embodiment 31, wherein the one or more filters are applied to a magnitude and an unwrapped angle of each extracted range bin from a range profile from each receiving antenna used to collect the radar data.
Embodiment 33: A heart monitoring system using a heart rate prediction model, the heart rate prediction model comprising:
   an input layer configured to receive input data comprising or derived from radar data;
   one or more two-dimensional (2-D) ResNet blocks;
   one or more one-dimensional (1-D) ResNet blocks; and
   an output layer configured to predict a heart rate.

## Claims

1. A computer-implemented method for transferring a machine-learned model trained on a first dataset of a first type of radar data to a second dataset of a second type of radar data to leverage learnings from the first and second datasets, the first dataset being larger than the second dataset, the computer-implemented method comprising:
(a) initializing the machine-learned model;
(b) training the machine-learned model on the first dataset of the first type of radar data and biometric ground truth data;
after (b), then (c) training the machine-learned model on the second dataset of the second type of radar data and the biometric ground truth data; and
after (c), then (d) outputting the machine-learned model for use in predicting one or more biometric parameters of a user from the radar data.

2. The computer-implemented method of claim 1, wherein the first dataset of the first type of radar data comprises paired mm-wave Frequency Modulated Continuous Wave, FMCW, radar data.

3. The computer-implemented method of claim 1 or 2, wherein the second dataset of the second type of radar data comprises paired impulse-radio ultra-wideband, IR-UWB, radar data.

4. The computer-implemented method of any one of claims 1 to 3, wherein the biometric ground truth data comprises at least one of electrocardiogram, ECG, signals or photoplethysmogram, PPG, signals.

5. The computer-implemented method of any one of the preceding claims, further comprising collecting at least one of the first dataset of the first type of radar data or the second dataset of the second type of radar data using one or more mobile computing devices.

6. The computer-implemented method of claim 5, wherein the one or more mobile computing devices comprises at least one of a smartphone, a smart display, a tablet, a laptop, a desktop, or a wearable computing device.

7. The computer-implemented method of any one of the preceding claims, wherein the machine-learned model comprises at least one of one or more two-dimensional ResNet blocks or one or more one-dimensional ResNet blocks.

8. The computer-implemented method of claim 7, wherein the machine-learned model comprises the one or more two-dimensional ResNet blocks followed by the one or more one-dimensional ResNet blocks.

9. The computer-implemented method of any one of the preceding claims, wherein the one or more biometric parameters of the user comprises a heart rate signal.

10. The computer-implemented method of any one of the preceding claims, further comprising pre-processing at least one of the first dataset of the first type of radar data or the second dataset of the second type of radar data before training the machine-learned model.

11. The computer-implemented method of claim 10, wherein pre-processing at least one of the first dataset of the first type of radar data or the second dataset of the second type of radar data further comprises applying one or more filters derived adaptively from a respiratory rate per sample to at least one of the first dataset of the first type of radar data or the second dataset of the second type of radar data to facilitate extraction of the heart rate signal.

12. The computer-implemented method of claim 11, wherein applying the one or more filters further comprises applying the one or more filters to a magnitude and an unwrapped angle of each extracted range bin from a range profile from each receiving antenna used to collect at least one of the first dataset of the first type of radar data or the second dataset of the second type of radar data.

13. Computer-readable media storing instructions which, when executed by at least one processor of a heart monitoring systems, cause the heart monitoring system to carry out a method of any one of claims 1 to 12.

14. A heart monitoring system, comprising at least one processor and a memory, the memory including instructions, which, when executed by the at least one processor, cause the heart monitoring system to carry out a method of any one of claims 1 to 12.

15. The heart monitoring system of claim 14, comprising one or more mobile computing devices for collecting least one of the first dataset of the first type of radar data or the second dataset of the second type of radar data.
